# EUROPEAN PATENT APPLICATION

(11) **EP 1 308 458 A1**
(43) Date of publication of application: **07.05.2003**
(21) Application number: 01402814.6
(22) Date of filing: 30.10.2001
(51) Int. Cl.: C07K 14/47, C12Q 1/68, C12N 15/85, C12N 5/10, C07K 16/18, C12N 15/10, G01N 33/574, A61K 38/17, A61K 48/00

(54) **New polynucleotides and polypeptides of the CX26 gene**

(71) Applicant: GenOdyssee, 91974 Courtaboeuf (FR)
(72) Inventor: Escary, Jean-Louis, 78150 Le Chesnay (FR)
(74) Representative: Santarelli

(57) **Abstract**

The present invention relates to new polynucleotides deriving from the nucleotide sequence of the gene CX26 and comprising a new SNP, new polypeptides derived from the natural protein Cx26 and comprising a mutation caused by this SNP as well as their therapeutic uses.

## Description

The present invention relates to new polynucleotides deriving from the nucleotide sequence of the CX26 gene and comprising a new SNP, new polypeptides derived from the natural protein Cx26 and comprising a mutation caused by this SNP as well as their therapeutic uses.

### PRIOR ART

The gene encoding for the "gap junction protein connexin 26" also known as "gap junction protein beta 2 (GJB2)", hereinafter referred to as "CX26", is well described in the prior art. The natural protein encoded by this gene is hereinafter referred to as "Cx26".

The nucleotide sequence of this gene is accessible under accession number AL138688.27 in the GenBank database.

A large number of subjects are affected by hearing impairment. In developed countries deafness has an important genetic origin and at least 60% of the cases are inherited. The pattern of inheritance can be dominant, recessive, X-linked and mitochondrial.

Many genes are involved in the different types of deafness (syndromic and non-syndromic). Non-syndromic hereditary deafness is mainly (80%) due to recessive genes (or mutations). It is believed that more than one hundred genes could be involved in hearing impairment. Several of these genes have been identified recently by positional cloning or positional candidate gene approaches.

Despite the fact that more than 20 loci have been described for non-syndromic autosomal recessive deafness (DFNB), a single locus, DFNB1, accounts for a high proportion of the cases, with variability depending on the population. The gene involved in this type of deafness is CX26, which encodes the gap junction protein connexin 26 (Cx26). Connexins are transmembrane proteins that form channels allowing rapid transport of ions or small molecules between cells. Connexins are expressed in many different tissues. Connexins are designated by their molecular mass (e.g. Cx26) or classified into two categories, alpha- and beta-gap junction proteins, respectively, according to sequence similarities at the nucleotide and amino acid levels (e.g. GJB2).

Many non-syndromic mutations, either dominant or recessive, have been identified in CX26.

Among the non-syndromic mutations described in CX26, 35delG (35delG mutation is also named 30delG because it corresponds to the deletion of one G within a stretch of 6 Gs at position 30 to 35) has been found to be common in most of the populations studied especially in the Mediterranean region.

Publications describing the 35delG mutations are :
- Carrasqillo et al. (1997). Two different connexin 26 mutations in an inbred kindred segregating non-syndromic recessive deafness: implications for genetic studies in isolated populations. Hum. Mol. Genet. 6:2163-2172.
- Denovelle et al. (1997). Prelingual deafness: high prevalence of a 30delG mutation in the connexin 26 gene. Hum. Mol. Genet. 6:2173-2177.
- Zelante et al. (1997). Connexin26 mutations associated with the most common form of non-syndromic neurosensory autosomal recessive deafness (DFNB1) in Mediterraneans. Hum. Mol. Genet. 6:1605-1609.
- Estivill et al. (1998). Connexin-26 mutations in sporadic and inherited sensorineural deafness. Lancet. 351:394-398.
- Kelley et al. (1998). Novel mutations in the connexin 26 gene (GJB2) that cause autosomal recessive (DFNB1) hearing loss. Am. J. Hum. Genet. 62:792-799.
- Lench et al. (1998). A Moroccan family with autosomal recessive sensorineural hearing loss caused by a mutation in the gap junction protein gene connexin 26 (GJB2). J. Med. Genet. 35:151-2.
- Lench et al. (1998). Connexin-26 mutations in sporadic non-syndromal sensorineural deafness Lancet. 351:415.
- Scott et al. (1998). Identification of mutations in the connexin 26 gene that cause autosomal recessive nonsyndromic hearing loss. Hum. Mutat. 11:5, 387-94.

An other mutation, 167delT, has been found to be common in patients of Jewish origin. Publications describing the recessive 167delT mutation are :
- Kelley et al. (1998). Novel mutations in the connexin 26 gene (GJB2) that cause autosomal recessive (DFNB1) hearing loss. Am. J. Hum. Genet. 62:792-9.
- Morell et al. (1998). Mutations in the connexin 26 gene (GJB2) among Ashkenazi Jews with nonsyndromic recessive deafness. N. Engl. J. Med. 339:1500-5.

An other mutation, 235delC, has been found to be common among the Asian population (Japanese). One publication describes this recessive mutation: Fuse et al. (1999). Three novel connexin26 gene mutations in autosomal recessive non-syndromic deafness NeuroReport. 10:1853-1857.

Several other mutations have been described in Cx26, most of them found in few individuals.

Three Cx26 mutations have been described to cause dominant deafness:
- M34T (Kelsell et al. (1997). Connexin 26 mutations in hereditary non-syndromic sensorineural deafness. Nature 387:80-3). M34T mutation has been found in heterozygosity in a family with few affected subjects (Kelsell et al. (1997)), but also in normal subjects (Kelley et al. (1998). Novel mutations in the connexin 26 gene (GJB2) that cause autosomal recessive (DFNB1) hearing loss. Am. J. Hum. Genet. 62:792-9 ; Scott et al. (1998). Connexin mutations and hearing loss. Nature. 391:32 ; Scott et al (1998). Identification of mutations in the connexin 26 gene that cause autosomal recessive nonsyndromic hearing loss. Hum. Mutat. 11:5, 387-94) and in a recessive family (Kelley et al. ;1998). It is believed that M34T is a dominant mutation with reduced penetrance. White et al. (1998) (Connexin mutations in deafness. Nature. 394, 630-1) performed functional studies on some mutations by expression in Xenopus Laevis oocytes, and showed that M34T had a dominant negative effect.
- W44C (Denovelle et al. (1998). Connexin 26 gene linked to a dominant deafness. Nature 393:319-20).
- W44S (Denovelle et al. (1998). Connexin 26 gene linked to a dominant deafness. Nature. 393:319-20).

Many syndromic deafness mutations have been identified in CX26. For example :
- R75W (Richard et al. ;1998). Functional defects of CX26 resulting from a heterozygous missense mutation in a family with dominant deaf-mutism and palmoplantar keratoderma. Hum. Genet. 103:393-9). Mutation R75W has been identified in two affected members of a family with deafness and diffuse keratoderma.
- D66H (Maestrini et al. ;1999). A missense mutation in connexin26, D66H, causes mutilating keratoderma with sensorineural deafness (Vohwinkel's syndrome) in three unrelated families. Hum. Mol. Genet. 8:1237-43). This missense mutation in connexin 26 has been identified in three families presenting sensorineural deafness and mutilating keratoderma. The mutation seems to be totally penetrant, as all patients in the three pedigrees presenting D66H were affected.

Another Cx26 mutation, G59A, has been recently identified in a family with dominant deafness and hyperkeratosis (Heathcote et al. (2000). A connexin 26 mutation causes a syndrome of sensorineural hearing loss and palmoplantar hyperkeratosis. J. Med. Genet. 37:50-51). All affected members of the family inherited the G59A mutation in GJB2. Another mutation in this gene, F83L was identified in only two subjects, one affected and one not.

Recently, Tanaka et al. demonstrated that the expression of Cx26 is greatly reduced or undetectable in human bladder and breast cancers (Tanaka et al. (2001). Connexin 26 enhances the bystander effect in HSVtk/GCV gene therapy for human bladder cancer by adenovirus/PLL/DNA gene delivery. Gene Therapy 8:139-148). The Cx26 protein is involved in gap junctional intercellular communication and is a putative tumor suppressor (Tanaka M. and Grossman H.B.(2001) "The 4th annual meeting of the American Society of Gene Therapy". May 30-June 3, 2001, Seattle, Washington).

It is known that the CX26 gene is involved in different human disorders and/or diseases, such as dominant and recessive deafness, non-syndromic autosomal or syndromic deafness, diffuse keratoderma, mutilating keratoderma, hyperkeratosis, bladder and breast cancers.

The inventor has found new polypeptides and new polynucleotides which are analogs to the CX26 gene capable of having a different functionality from the natural Cx26 protein.

These new polypeptides and polynucleotides can notably be used to treat or prevent the disorders or diseases previously mentioned.

### THE INVENTION

The applicant has identified new polynucleotides that differ from the nucleotide sequence of the reference wild-type CX26 gene, in that it comprises one new SNP (Single Nucleotide Polymorphism).

The nucleotide sequence SEQ ID N°1 of the human reference wild-type CX26 gene is composed of 13980 nucleotides and comprises a coding sequence of 681 nucleotides, from nucleotide 12681 (start codon) to the nucleotide 13361 (stop codon).

The applicant has identified one SNP in the nucleotide sequence of the reference wild-type CX26 gene : c12972t.

It is understood, in the sense of the present invention, that the numbering corresponding to the positioning of the SNP previously defined is relative to the numbering of the nucleotide sequence SEQ ID N°1.

The SNP c12972t of the nucleotide sequence SEQ ID N°1 corresponds to the SNP c292t positioned on the coding sequence of 681 nucleotides of the human reference wild-type CX26 gene.

The letters a, t, c and g correspond respectively to the nitrogenous bases adenine, thymine, cytosine and guanine of the nucleotide.

Thus, for example, the SNP c12972t corresponds to a substitution of the cytosine (c) wild-type nucleotide at position 12972 of the nucleotide sequence SEQ ID N°1, by a thymine (t) nucleotide.

This SNP has been identified by the applicant using the determination process described in applicant's patent application FR 00 22894, entitled " Procédé de détermination d'un ou plusieurs polymorphisme(s) fonctionnel(s) dans la séquence nucléique d'un gène "candidat" fonctionnel présélectionné et ses applications" (Process for the determination of one or several functional polymorphism(s) in the nucleotide sequence of a preselected functional candidate gene and its applications) and filed December 6, 2000, cited here by way of reference.

The process described in this patent application permits the identification of one (or several) preexisting SNP(s) in at least one individual from a random population of individuals.

In the scope of the present invention, a fragment of the nucleotide sequence of the CX26 gene, comprising, for example, the coding sequence, was isolated from different individuals in a population of individuals chosen in a random manner.

Sequencing of these fragments was then carried out on certain of these samples having a heteroduplex profile (that is a profile different from that of the reference wild-type CX26 gene sequence) after analysis by DHPLC ("Denaturing-High Performance Liquid Chromatography").

The fragment sequenced in this way was then compared to the nucleotide sequence of the fragment of the reference wild-type CX26 gene and the SNP in conformity with the invention identified.

Thus, the SNP c12972t is natural and is present in certain individuals of the world population such as South Americans (Andes).

The reference wild-type CX26 gene encodes for a protein of 226 amino acids, corresponding to the amino acid sequence SEQ ID N°2.

The SNP c12972t causes a mutation of the amino acid arginine (R) at position 98 in the protein of the gene CX26, corresponding to the amino acid sequence SEQ ID N°2, in tryptophane (W).

It is understood, in the sense of the present invention, that the numbering corresponding to the positioning of the R98W is relative to the numbering of the amino acid sequence SEQ ID N°2.

It appears that arginine (R) at position 98 is conserved on most Cx26 protein orthologues, including mouse and rat Cx26, but not in sheep where a tyrosine (Y98, an aromatic residue) is found at this position.

Regarding human Cx26 paralogues, arginine 98 (R98) is found in Cx30.3, Cx31, Cx31.1, and Cx59. Nevertheless, one finds a glutamine (Q98) in Cx37, Cx43, Cx46, Cx30 and Cx50, but no tryptophane (W) has ever been observed.

Beside the fact that tryptophane is a rare residue, this amino acid bears particular physico-chemical properties associated with its hydrophobic lateral cycle, properties in opposition with those of arginine, the later being also polar but positively charged. The difference between Q and W amino acids lies in the fact that glutamine (Q) is a polar residue whereas tryptophan (W) is a slightly hydrophobic one with indole ring.

From a structural point of view, Cx26 is a protein with 3 to 4 transmembrane domains. The R98 amino acid is located immediately after the second of these transmembrane domains, facing the intracellular side of the membrane.

Cx26 is a short protein (226 amino acid long). Many CX26 mutations have been reported associated with deafness, and interestingly enough, these mutations are scattered all over the protein sequence. More, many mutations have been reported nearby position 98. Many of these have been associated with recessive deafness, for example L90P, V95M, H100Y, and S113R.

Because of physico-chemical changes due to the R98W variation, and the apparent conservation of an arginine at position 98, it is very likely that the W98 Cx26 variant will be impaired in its function.

The invention also has for an object the use of polynucleotides and of polypeptides in conformity with the invention as well as of therapeutic molecules obtained and/or identified starting from these polynucleotides and polypeptides, notably for the prevention and the treatment of certain human disorders and/or diseases.

### DETAILED DESCRIPTION OF THE INVENTION

### Definitions

By "nucleotide sequence of the reference wild-type gene" is understood the nucleotide sequence SEQ ID N° 1.

This sequence is accessible in GenBank under Accession number AL138688.27.

By "natural Cx26 protein" is understood the protein encoded by the nucleotide sequence of the reference wild-type CX26 gene.

By "polynucleotide" is understood a polyribonucleotide or a polydeoxyribonucleotide that can be a modified or non-modified DNA or an RNA.

The term polynucleotide includes, for example, a single strand or double strand DNA, a DNA composed of a mixture of one or several single strand region(s) and of one or several double strand region(s), a single strand or double strand RNA and an RNA composed of a mixture of one or several single strand region(s) and of one or several double strand region(s). The term polynucleotide can also include an RNA and/or a DNA including one or several triple strand regions. By polynucleotide is equally understood the DNAs and RNAs containing one or several bases modified in such a fashion as to have a skeleton modified for reasons of stability or for other reasons. By modified base is understood, for example, the unusual bases such as inosine.

By "polypeptide" is understood a peptide, an oligopeptide, an oligomer or a protein comprising at least two amino acids joined to each other by a normal or modified peptide bond, such as in the cases of the isosteric peptides, for example.

A polypeptide can be composed of amino acids other than the 20 amino acids defined by the genetic code. A polypeptide can equally be composed of amino acids modified by natural processes, such as post translational maturation processes or by chemical processes, which are well known to a person skilled in the art. Such modifications are fully detailed in the literature. These modifications can appear anywhere in the polypeptide: in the peptide skeleton, in the amino acid chain or even at the carboxy- or amino-terminal ends.

A polypeptide can be branched following an ubiquitination or be cyclic with or without branching. This type of modification can be the result of natural or synthetic post-translational processes that are well known to a person skilled in the art.

For example, by polypeptide modifications is understood acetylation, acylation, ADP-ribosylation, amidation, covalent fixation of flavine, covalent fixation of heme, covalent fixation of a nucleotide or of a nucleotide derivative, covalent fixation of a lipid or of a lipidic derivative, the covalent fixation of a phosphatidylinositol, covalent or non-covalent cross-linking, cyclization, disulfide bridge formation, demethylation, cysteine formation, pyroglutamate formation, formylation, gamma-carboxylation, glycosylation, GPI anchor formation, hydroxylation, iodization, methylation, myristoylation, oxidation, proteolytic processes, phosphorylation, prenylation, racemization, seneloylation, sulfatation, amino acid addition such as arginylation or ubiquitination. Such modifications are fully detailed in the literature: PROTEINS-STRUCTURE AND MOLECULAR PROPERTIES, 2^{nd} Ed., T. E. Creighton, New York, 1993, POST-TRANSLATIONAL COVALENT MODIFICATION OF PROTEINS, B. C. Johnson, Ed., Academic Press, New York, 1983, Seifter et al. "Analysis for protein modifications and nonprotein cofactors", Meth. Enzymol. (1990) 182:626-646 et Rattan et al. "Protein Synthesis: Post-translational Modifications and Aging", Ann NY Acad Sci (1992) 663: 48-62.

By "isolated polynucleotide" or "isolated polypeptide" is understood a polynucleotide or a polypeptide such as previously defined which is isolated from the human body or otherwise produced by a technical process.

By "identity" is understood the measurement of nucleotide or polypeptide sequence identity.

Identity is a term well known to a person skilled in the art and well described in the literature. See COMPUTATIONAL MOLECULAR BIOLOGY, Lesk, A.M., Ed., Oxford University Press, New York, 1998; BIOCOMPUTING INFORMATICS AND GENOME PROJECT, Smith, D.W., Ed., Academic Press, New York, 1993; COMPUTER ANALYSIS OF SEQUENCE DATA, PART I, Griffin, A.M. and Griffin H.G., Ed, Humana Press, New Jersey, 1994; et SEQUENCE ANALYSIS IN MOLECULAR BIOLOGY, von Heinje, G., Academic Press, 1987.

The methods commonly employed to determine the identity and the similarity between two sequences are equally well described in the literature. See GUIDE TO HUGE COMPUTER, Martin J. Bishop, Ed, Academic Press, San Diego, 1994, et Carillo H. and Lipton D., Siam J Applied Math (1988) 48:1073.

A polynucleotide having, for example, an identity of at least 95 % with the nucleotide sequence SEQ ID N° 1 is a polynucleotide which contains at most 5 points of mutation over 100 nucleotides, compared to said sequence.

These points of mutation can be one (or several) substitution(s), addition(s) and/or deletion(s) of one (or several) nucleotide(s).

In the same way, a polypeptide having, for example, an identity of at least 95 % with the amino acid sequence SEQ ID N° 2 is a polypeptide that contains at most 5 points of mutation over 100 amino acids, compared to said sequence.

These points of mutation can be one (or several) substitution(s), addition(s) and/or deletion(s) of one (or several) amino acid(s).

The polynucleotides and the polypeptides according to the invention which are not totally identical with respectively the nucleotide sequence SEQ ID N°1 or the amino acid sequence SEQ ID N°2, (it being understood that these sequences SEQ ID N°1 and SEQ ID N°2 contain the SNP of the invention) are considered as variants of these sequences.

Usually a polynucleotide according to the invention possesses the same or practically the same biological activity as the nucleotide sequence SEQ ID N°1 comprising the SNP of the invention.

In similar fashion, usually a polypeptide according to the invention possesses the same or practically the same biological activity as the amino acid sequence SEQ ID N°2 comprising the coding SNP of the invention.

A variant, according to the invention, can be obtained, for example, by site-directed mutagenesis or by direct synthesis.

By "SNP" is understood any natural variation of a base in a nucleotide sequence. A SNP, on a nucleotide sequence, can be coding, silent or non-coding.

A coding SNP is a polymorphism included in the coding sequence of a nucleotide sequence that involves a modification of an amino acid in the sequence of amino acids encoded by this nucleotide sequence. In this case, the term SNP applies equally, by extension, to a mutation in an amino acid sequence.

A silent SNP is a polymorphism included in the coding sequence of a nucleotide sequence that does not involve a modification of an amino acid in the amino acid sequence encoded by this nucleotide sequence.

A non-coding SNP is a polymorphism included in the non-coding sequence of a nucleotide sequence. This polymorphism can notably be found in an intron, a splicing zone, a transcription promoter or an enhancer site sequence.

By "functional SNP" is understood a SNP, such as previously defined, which is included in a nucleotide sequence or an amino acid sequence, having a functionality.

By "functionality" is understood the biological activity of a polypeptide or, by extension, of a polynucleotide coding for said polypeptide.

The functionality of a polypeptide or of a polynucleotide according to the invention can consist in a conservation, an augmentation, a reduction or a suppression of the biological activity of the polypeptide encoded by the nucleotide sequence of the reference wild-type gene or of this latter nucleotide sequence.

The functionality of a polypeptide or of a polynucleotide according to the invention can equally consist in a change in the nature of the biological activity of the polypeptide encoded by the nucleotide sequence of the reference wild-type gene or of this latter nucleotide sequence.

The biological activity can, notably, be linked to the affinity or to the absence of affinity of a polypeptide according to the invention with a receptor.

### Polynucleotide

The present invention has for its first object an isolated polynucleotide comprising:
a) a nucleotide sequence having at least 80 % identity, preferably at least 90 % identity, more preferably at least 95 % identity and still more preferably at least 99 % identity with the sequence SEQ ID N° 1 or its coding sequence (of the nucleotide 12681 to the nucleotide 13361), it being understood that this nucleotide sequence comprises the coding SNP c12972t; or
b) a nucleotide sequence complementary to a nucleotide sequence under a).

The present invention relates equally to an isolated polynucleotide comprising:
a) the nucleotide sequence SEQ ID N° 1 or its coding sequence, it being understood that each of these sequences comprises the coding SNP: c12972t; or
b) a nucleotide sequence complementary to a nucleotide sequence under a).

Preferably, the polynucleotide of the invention consists of the sequence SEQ ID N° 1 or its coding sequence, it being understood that each of these sequences comprises the coding SNP : c12972t.

The present invention concerns also an isolated polynucleotide consisting of a part of a polynucleotide according to the invention it being understood that the sequence of said isolated polynucleotide contains the coding SNP of the invention : c12972t, said isolated polynucleotide being composed of at least 10 nucleotides.

Preferably, the isolated polynucleotide as defined above is composed of 10 to 40 nucleotides.

The present invention also has for its object an isolated polynucleotide coding for a polypeptide comprising the amino acid sequence SEQ ID N° 2, it being understood that the amino acid sequence comprises the coding SNP : R98W.

Preferably a polynucleotide according to the invention is composed of a DNA or RNA molecule.

A polynucleotide according to the invention can be obtained by standard DNA or RNA synthetic methods.

A polynucleotide according to the invention, comprising the SNP c12972t, can be obtained by site-directed mutagenesis starting from the nucleotide sequence of the gene CX26 by modifying the nucleotide cytosine (c) by the nucleotide thymine (t) at position 12972 on the nucleotide sequence SEQ ID N°1.

The processes of site-directed mutagenesis that can be implemented in this way are well known to a person skilled in the art. The publication of TA Kunkel in 1985 in "Proc. Natl. Acad. Sci. USA" 82:488 can notably be mentioned.

An isolated polynucleotide can equally include, for example, nucleotide sequences coding for pre-, pro- or pre-pro-protein amino acid sequences or marker amino acid sequences, such as hexa-histidine peptide.

A polynucleotide of the invention can equally be associated with nucleotide sequences coding for other proteins or protein fragments in order to obtain fusion proteins or other purification products.

A polynucleotide according to the invention can equally include nucleotide sequences such as the 5' and/or 3' non-coding sequences, such as, for example, transcribed or non-transcribed sequences, translated or non-translated sequences, splicing signal sequences, polyadenylated sequences, ribosome binding sequences or even sequences which stabilize mRNA.

A nucleotide sequence complementary to the nucleotide or polynucleotide sequence is defined as one that can be hybridized with this nucleotide sequence, under stringent conditions.

By "stringent hybridization conditions" is generally but not necessarily understood the chemical conditions that permit a hybridization when the nucleotide sequences have an identity of at least 80 %, preferably at least 90%, preferably greater than or equal to 95 %, still more preferably greater than or equal to 97 % and most preferably greater than or equal to 99 %, such as 100%.

The stringent conditions can be obtained according to methods well known to a person skilled in the art and, for example, by an incubation of the polynucleotides, at 42° C, in a solution comprising 50 % formamide, 5xSSC (150 mM of NaCI, 15 mM of trisodium citrate), 50 mM of sodium phosphate (pH = 7.6), 5x Denhardt Solution, 10 % dextran sulfate and 20 µg denatured salmon sperm DNA, followed by washing the filters at 0.1x SSC, at 65° C.

Within the scope of the invention, when the stringent hybridization conditions permit hybridization of the nucleotide sequences having an identity equal to 100 %, the nucleotide sequence is considered to be strictly complementary to the nucleotide sequence such as described under a).

It is understood within the meaning of the present invention that the nucleotide sequence complementary to a nucleotide sequence comprises the anti-sense SNP according to the invention.

Thus, as the nucleotide sequence comprises the SNP c12972t, its complementary nucleotide sequence still comprises the adenine (a) nucleotide at position 12972.

### Identification, hybridization and/or amplification of a polynucleotide comprising a SNP

The present invention also has for its object the use of all or part of a previously defined polynucleotide, in order to identify, hybridize and/or amplify all or part of a polynucleotide consisting of the nucleotide sequence SEQ ID N° 1 or of its coding sequence (of the nucleotide 12681 to the nucleotide 13361), it being understood that each one of these sequences comprises at least the SNP: c12972t.

### Genotyping and determination of the frequency of a SNP

The present invention equally has for its object the use of all or part of a polynucleotide according to the invention for the genotyping of a nucleotide sequence which has 90 to 100 % identity (at least 90%, preferably 95%, more preferably 97% and particularly 100%) with the nucleotide sequence of CX26 gene and which comprises the SNP : c12972t.

According to the invention, the genotyping may be carried out on an individual or a population of individuals.

Within the meaning of the invention, genotyping is defined as a process for the determination of the genotype of an individual or of a population of individuals. Genotype consists of the alleles present at one or more specific loci.

By "population of individuals" is understood a group of determined individuals selected in random or non-random fashion. These individuals can be humans, animals, microorganisms or plants.

Usually, the group of individuals comprises at least 10 persons, preferably from 100 to 300 persons.

The individuals can be selected according to their ethnicity or according to their phenotype, notably those who are affected by the following disorders and/or diseases : dominant and recessive deafness, non-syndromic autosomal or syndromic deafness, diffuse keratoderma, mutilating keratoderma, hyperkeratosis, bladder and breast cancers.

A functional SNP according to the invention is preferably genotyped in a population of individuals.

Multiple technologies exist which can be implemented in order to genotype SNPs (see notably Kwok Pharmacogenomics, 2000, vol 1, pp 95-100. "High-throughput genotyping assay approaches"). These technologies are based on one of the four following principles: allele specific oligonucleotide hybridization, oligonucleotide elongation by dideoxynucleotides optionally in the presence of deoxynucleotides, ligation of allele specific oligonucleotides or cleavage of allele specific oligonucleotides. Each one of these technologies can be coupled to a detection system such as measurement of direct or polarized fluorescence, or mass spectrometry.

Genotyping can notably be carried out by minisequencing with hot ddNTPs (2 different ddNTPs labeled by different fluorophores) and cold ddNTPs (2 non labeled ddNTPs), in connection with a polarized fluorescence scanner. The minisequencing protocol with reading of polarized fluorescence (Technology FP-TDI or Fluorescence Polarization Template-direct Dye-Terminator Incorporation) is well known to a person skilled in the art.

It can be carried out on a product obtained after amplification by polymerase chain reaction (PCR) of the DNA of each individual. This PCR product is selected to cover the polynucleotide genic region containing the studied SNP. After the last stage in the PCR thermocycler, the plate is then placed on a polarized fluorescence scanner for a reading of the labeled bases by using fluorophore specific excitation and emission filters. The intensity values of the labeled bases are reported on a graph.

The sense and antisense primers respectively for the PCR amplification, in the case of a SNP of the invention, can easily be selected by a person skilled in the art according to the position of the SNP according to the invention.

For example, the sense and antisense nucleotide sequences for the PCR amplification can be respectively SEQ ID N° 3 and SEQ ID N° 4.

The nucleotide sequences permit amplification of a fragment having a length of 345 nucleotides, of the nucleotide 12662 to the nucleotide 13006 in the nucleotide sequence SEQ ID N° 1.

A statistical analysis of the frequency of each allele (allelic frequency) encoded by the gene comprising the SNP in the population of individuals is then achieved, which permits determination of the importance of their impact and their distribution in the different sub-groups and notably, if necessary, the diverse ethnic groups that constitute this population of individuals.

The genotyping data are analyzed in order to estimate the distribution frequency of the different alleles observed in the studied populations. The calculations of the allelic frequencies can be carried out with the help of software such as SAS-suite® (SAS) or SPLUS® (MathSoft). The comparison of the allelic distributions of a SNP of the invention across different ethnic groups of the population of individuals can be carried out by means of the software ARLEQUIN® and SAS-suite®.

### Expression vector and host cell

The present invention also has for its object a recombinant vector comprising at least one polynucleotide according to the invention.

Numerous expression systems can be used, like, for example, chromosomes, episomes, derived viruses. More particularly, the recombinant vectors used can be derived from bacterial plasmids, transposons, yeast episome, insertion elements, yeast chromosome elements, viruses such as baculovirus, papilloma viruses such as SV40, vaccinia viruses, adenoviruses, fox pox viruses, pseudorabies viruses, retroviruses.

These recombinant vectors can equally be cosmid or phagemid derivatives. The nucleotide sequence can be inserted in the recombinant expression vector by methods well known to a person skilled in the art such as, for example, those that are described in MOLECULAR CLONING, A LABORATORY MANUAL (supra) Sambrook et al.

The recombinant vector can include nucleotide sequences that control the regulation of the polynucleotide expression as well as nucleotide sequences permitting the expression and the transcription of a polynucleotide of the invention and the translation of a polypeptide of the invention, these sequences being selected according to the host cells that are used.

Thus, for example, an appropriate secretion signal can be integrated in the recombinant vector so that the polypeptide, encoded by the polynucleotide of the invention, will be directed towards the opening of the endoplasmic reticulum, towards the periplasmic space, on the membrane or towards the extracellular environment.

The present invention also has for its object a host cell comprising a recombinant vector according to the invention.

The introduction of the recombinant vector in a host cell can be carried out according to methods that are well known to a person skilled in the art such as those described in BASIC METHODS IN MOLECULAR BIOLOGY, Davis et al., 1986 et MOLECULAR CLONING: A LABORATORY MANUAL, 2^{nd} Ed., Cold Spring Harbor Laboratory Press, Cold Spring Harbor, N.Y., 1989, such as transfection by calcium phosphate, transfection by DEAE dextran, transfection, microinjection, transfection by cationic lipids, electroporation, transduction or infection.

The host cell can be, for example, bacterial cells such as cells of streptococci, staphylococci, *E. coli* or *Bacillus subtilis,* cells of fungi such as yeast cells and cells of *Aspergillus, Streptomyces,* insect cells such as cells of *Drosophilia* S2 and of *Spodoptera* Sf9, animal cells, such as CHO, COS, HeLa, C127, BHK, HEK 293 cells and human cells of the subject to treat or even plant cells.

The host cells can be used, for example, to express a polypeptide of the invention or as active product in pharmaceutical compositions, as will be seen hereinafter.

### Polypeptide

The present invention also has for its object an isolated polypeptide comprising an amino acid sequence having at least 80 % identity, preferably at least 90 % identity, more preferably at least 95 % identity and still more preferably at least 99 % identity with the amino acid sequence SEQ ID N° 2, it being understood that the amino acid sequence contains the coding SNP: R98W.

The polypeptide of the invention can equally comprise the amino acid sequence SEQ ID N° 2, it being understood that the amino acid sequence contains the following coding SNP: R98W.

The polypeptide of the invention can more particularly consist of the amino acid sequence SEQ ID N° 2, it being understood that the amino acid sequence contains the following coding SNP: R98W.

The present invention equally has for its object a process for the preparation of the above-described polypeptide, in which a previously defined host cell is cultivated in a culture medium and said polypeptide is isolated from the culture medium.

The polypeptide can be purified starting from the host cells, according to methods well known to a person skilled in the art such as precipitation with the help of chaotropic agents such as salts, in particular ammonium sulfate, ethanol, acetone or trichloroacetic acid, acid extraction; ion exchange chromatography; phosphocellulose chromatography; hydrophobic interaction chromatography; affinity chromatography; hydroxyapatite chromatography or exclusion chromatographies.

By "culture medium" is understood the medium in which the polypeptide of the invention is isolated or purified. This medium can be composed of the extracellular medium and/or the cellular lysate. Techniques well known to a person skilled in the art equally permit the latter to give back an active conformation to the polypeptide, if the conformation of said polypeptide was altered during the isolation or the purification.

### Antibodies

The present invention also has for its object a process for obtaining an immunospecific antibody.

By "antibody" is understood the monoclonal, polyclonal, chimeric, simple chain, humanized antibodies as well as the Fab fragments, including Fab or immunoglobulin expression library products.

An immunospecific antibody can be obtained by immunization of an animal with a polypeptide according to the invention.

The invention also relates to an immunospecific antibody for a polypeptide according to the invention, such as defined previously.

A polypeptide according to the invention, one of its fragments, an analog, one of its variants or a cell expressing this polypeptide can also be used to produce immunospecific antibodies.

The term "immunospecific" means that the antibody possesses a better affinity for the polypeptide of the invention than for other polypeptides known in the prior art.

The immunospecific antibodies can be obtained by administration of a polypeptide of the invention, of one of its fragments, of an analog or of an epitopic fragment or of a cell expressing this polynucleotide in a mammal, preferably non human, according to methods well known to a person skilled in the art.

For the preparation of monoclonal antibodies, typical methods for antibody production can be used, starting from cell lines, such as the hybridoma technique (Kohler et al., Nature (1975) 256: 495-497), the trioma technique, the human B cell hybridoma technique (Kozbor et al., Immunology Today (1983) 4: 72) and the EBV hybridoma technique (Cole et al., MONOCLONAL ANTIBODIES AND CANCER THERAPY, pp. 77-96, Alan R. Liss, 1985).

The techniques of single chain antibody production such as described, for example, in US Patent N° 4,946, 778 can equally be used.

Transgenic animals such as mice, for example, can equally be used to produce humanized antibodies.

### Agents interacting with the polypeptide of the invention

The present invention equally has for its object a process for the identification of an agent activating or inhibiting a polypeptide according to the invention, comprising:
a) the preparation of a recombinant vector comprising a polynucleotide according to the invention containing the coding SNP of the invention,
b) the preparation of host cells comprising a recombinant vector according to a),
c) the contacting of host cells according to b) with an agent to be tested, and
d) the determination of the activatory or inhibitory effect generated by the agent to test.

A polypeptide according to the invention can also be employed for a process for screening compounds that interact with it.

These compounds can be activating (agonists) or inhibiting (antagonists) agents of intrinsic activity of a polypeptide according to the invention. These compounds can equally be ligands or polypeptide substrates of the invention. See Coligan et al., Current Protocols in Immunology 1 (2), Chapter 5 (1991).

In general, in order to implement such a process, it is first desirable to produce appropriate host cells that express a polypeptide according to the invention. Such cells can be, for example, cells of mammals, yeasts, insects such as *Drosophilia* or bacteria such as *E. coli.*

These cells or membrane extracts of these cells are then put in the presence of compounds to be tested.

The linking capacity of the compounds to be tested with the polypeptide of the invention can then be observed, as well as the inhibition or the activation of the functional response.

Stage d) of the above process can be implemented by using a labeled agent to be tested directly or indirectly. It can also include a competition test, by using a labeled or non-labeled agent and a labeled competitor agent.

It can equally be determined if an agent to be tested leads to the generation of an activation or inhibition signal on cells expressing the polypeptide of the invention, by using appropriate detection means, following the signal to be detected.

Such activatory or inhibitory agents can be polynucleotides, and in certain cases oligonucleotides or polypeptides, such as proteins or antibodies, for example.

The present invention also has for its object a process for the identification of an agent activated or inhibited by a polypeptide according to the invention, comprising:
a) the preparation of a recombinant vector comprising a polynucleotide according to the invention containing the coding SNP of the invention,
b) the preparation of host cells comprising a recombinant vector according to a),
c) the contacting of host cells according to b) in the presence of an agent to be tested, and
d) the determination of the activatory or inhibitory effect generated by said polypeptide on the agent to be tested.

An agent activated or inhibited by the polypeptide of the invention is an agent that responds, respectively, to an activation or an inhibition in the presence of this polypeptide. The agents, activated or inhibited directly or indirectly by the polypeptide of the invention, can consist of polypeptides such as, for example, membranar or nuclear receptors, kinases and more preferably tyrosine kinases, transcription factor or polynucleotides.

### Detection of diseases

The present invention also has for its object a process for the detection of the expression of a polypeptide of amino acid sequence SEQ ID N°2 encoded by a polynucleotide according to the invention and/or a polypeptide according to the invention, comprising:
a) the detection of the presence or of the absence of a polynucleotide according to the invention in the genome of the subject, and/or
b) the determination of the concentration of a polypeptide according to the invention in a subject.

The detection of a polynucleotide and/or the determination of the concentration of a polypeptide according to the invention can permit one to know if a subject is affected or at risk of being affected or, to the contrary, presents a partial resistance to the development of a disease, of an indisposition or of a disorder such as previously defined, relative to the expression of a polypeptide of the invention.

Preferably, in stage a) of the detection process mentioned above, the presence or absence of a polynucleotide containing at least one coding SNP such as previously defined is going to be detected.

The detection of the polynucleotide can be carried out starting from biological samples of the subject to be studied, such as cells, blood, urine, saliva, or starting from a biopsy or an autopsy of the subject to be studied. The genomic DNA can be used for the detection directly or after a PCR amplification, for example. RNA or cDNA can equally be used in a similar fashion.

It is then possible to compare the nucleotide sequence of a polynucleotide according to the invention with the nucleotide sequence detected in the genome of the subject.

The comparison of the nucleotide sequences can be carried out by sequencing, by DNA hybridization methods, by mobility difference of the DNA fragments on an electrophoresis gel with or without denaturing agents or by fusion temperature difference. See Myers et al., Science (1985) 230:1242. Such modifications in the structure of the nucleotide sequence at a precise point can equally be revealed by nuclease protection tests, such as RNase and the S1 nuclease or also by chemical cleaving agents. See Cotton et al., Proc. Nat. Acad. Sci. USA (1985) 85: 4397-4401. Oligonucleotide probes comprising a polynucleotide fragment of the invention can equally be used to conduct the screening.

Numerous methods well known to a person skilled in the art can be used to determine the expression of a polynucleotide of the invention and to identify the genetic variability of this polynucleotide. See Chee et al., Science (1996), Vol 274, pp 610-613.

The present invention equally has for its object the use of a polynucleotide according to the invention to carry out a genetic diagnosis of a disease or of a resistance to a disease linked to the presence, in one or several individuals of the human population, of the mutant allele encoded by a functional SNP according to the invention.

These diseases can be disorders and/or human diseases, such as dominant and recessive deafness, non-syndromic autosomal or syndromic deafness, diffuse keratoderma, mutilating keratoderma, hyperkeratosis, bladder and breast cancers.

The determination of the polypeptide concentration according to the invention, in stage b), can equally help in the diagnosis of a disease, an indisposition or a disorder or, to the contrary, the resistance to a disease, an indisposition or a disorder in a subject by withdrawing a sample derived from that subject.

The increase or the decrease of the expression of the polypeptide can be measured by quantifying the level of RNA coding for this polypeptide, following methods well known to a person skilled in the art, for example, by PCR, RT-PCR, RNase protection, Northern blot, and other hybridization methods.

It is equally possible to determine the polypeptide concentration of the invention present in a biological sample of the subject by well known methods, for example, by radioimmunoassay, competitive binding tests, Western blot and ELISA tests.

Consecutively to stage b), the determined polypeptide concentration according to the invention can be compared with the natural protein concentration usually found in a subject.

A person skilled in the art can identify the threshold above or below which appears the sensitivity or, to the contrary, the resistance to the disease, the indisposition or the disorder evoked above, with the help of prior art publications or by conventional tests or assays, such as those that are previously mentioned.

### Medications and treatments of diseases

The present invention also has for its object a medication containing as an active agent, a polypeptide according to the invention.

The invention also relates to the use of a polypeptide according to the invention, for the preparation of a medication intended for the prevention or the treatment of different human disorders and/or diseases, such as dominant and recessive deafness, non-syndromic autosomal or syndromic deafness, diffuse keratoderma, mutilating keratoderma, hyperkeratosis, bladder and breast cancers.

Certain of the compounds permitting to obtain the polypeptide according to the invention as well as the compounds obtained or identified starting from this polypeptide can likewise be used for the therapeutic treatment of the human body, i.e., by way of medication.

This is why the present invention also has for an object a medication containing, by way of active agent a polynucleotide according to the invention containing the previously defined coding SNP, a previously defined recombinant vector, a previously defined host cell and/or a previously defined antibody.

The invention also relates to the use of a polynucleotide according to the invention containing at least the previously defined coding SNP, a previously defined recombinant vector, a previously defined host cell and/or a previously defined antibody, for the preparation of a medication intended for the prevention or the treatment of different human disorders and/or diseases, such as dominant and recessive deafness, non-syndromic autosomal or syndromic deafness, diffuse keratoderma, mutilating keratoderma, hyperkeratosis, bladder and breast cancers.

The dosage of a polypeptide and of the other compounds of the invention, useful as active agent, depends on the choice of the compound, the therapeutic indication, the mode of administration, the nature of the formulation, the nature of the subject and the judgment of the doctor.

When it is used as active agent, a polypeptide according to the invention is generally administered at doses ranging between 1 and 100 µg/kg of the subject, per day and according to the mode of administration.

The invention also has as an object a pharmaceutical composition that contains at least one above-mentioned compound, such as a polypeptide according to the invention, all or part of a polynucleotide according to the invention containing at least one previously defined coding SNP, a previously defined recombinant vector, a previously defined host cell and/or a previously defined antibody, by way of active agent, as well as a pharmaceutically acceptable excipient.

In these pharmaceutical compositions, the active agent is advantageously present at physiologically effective doses.

These pharmaceutical compositions can be, for example, solids or liquids and be present in pharmaceutical forms currently used in human medicine, such as for example simple or coated tablets, gelcaps, granules, caramels, suppositories and preferably injectable preparations and powders for injectables. These pharmaceutical forms can be prepared according to typical methods.

The active agent(s) can be incorporated into typically employed excipients in these pharmaceutical compositions, such as talc, Arabic gum, lactose, starch, dextrose, glycerol, ethanol, magnesium stearate, cocoa butter, aqueous or non-aqueous vehicles, fatty substances of animal or vegetable origin, paraffinic derivatives, glycols, various wetting agents, dispersants or emulsifiers, preservatives.

The active agent(s) according to the invention can be employed alone or in combination with other compounds, such as therapeutic compounds.

The different formulations of the pharmaceutical compositions are adapted according to the mode of administration.

The pharmaceutical compositions can be administered by different routes of administration known to a person skilled in the art.

The invention equally has for an object a diagnostic composition that contains at least one above-mentioned compound, such as a polypeptide according to the invention, all or part of a polynucleotide according to the invention, a previously defined recombinant vector, a previously defined host cell and/or a previously defined antibody.

This diagnostic composition may contain, for example, an appropriate excipients generally used in the diagnostic composition such as buffers and preservatives.

The present invention equally has as an object the use:
a) of a therapeutically effective quantity of a polypeptide according to the invention, and/or
b) of a polynucleotide according to the invention, and/or
c) of a host cell of the subject to be treated, previously defined, to prepare a medication intended to increase the expression or the activity, in a subject, of a polypeptide according to the invention.

Thus, to treat a subject who needs an increase in the expression or in the activity of a polypeptide of the invention, several methods are possible.

It is also possible to administer to the subject a therapeutically effective quantity of a polypeptide of the invention and/or of the activatory agent and/or activated such as previously defined, possibly in combination, with a pharmaceutically acceptable excipient.

It is likewise possible to increase the endogenous production of a polypeptide of the invention by administration to the subject of a polynucleotide according to the invention. For example, this polynucleotide can be inserted in a retroviral expression vector. Such a vector can be isolated starting from cells having been infected by a retroviral plasmid vector containing RNA encoding for the polypeptide of the invention, in such a fashion that the transduced cells produce infectious viral particles containing the gene of interest. See Gene Therapy and other Molecular Genetic-based Therapeutic Approaches, Chapter 20, in Human Molecular Genetics, Strachan and Read, BIOS Scientifics Publishers Ltd (1996).

It is equally possible to administer to the subject host cells belonging to him, these host cells having been taken and modified, preliminarily, so as to express the polypeptide of the invention, as previously described.

The present invention equally relates to the use:
a) of a therapeutically effective quantity of a previously defined immunospecific antibody, and/or
b) of a polynucleotide permitting inhibition of the expression of a polynucleotide according to the invention,

In order to prepare a medication intended to reduce the expression or the activity, in a subject, of a polypeptide according to the invention.

It is also possible to administer to the subject a therapeutically effective quantity of an inhibitory agent and/or of an antibody such as previously defined, possibly in combination, with a pharmaceutically acceptable excipient.

It is equally possible to reduce the endogenous production of a polypeptide of the invention by administration to the subject of a complementary polynucleotide according to the invention permitting inhibition of the expression of a polynucleotide of the invention.

The present invention concerns also the use of a Cx26 protein for the preparation of a medication for the prevention or the treatment of a patient having a disorder or a disease caused by the R98W Cx26 variant linked to the presence in the genome of said patient of a nucleotide sequence having at least 95% identity (preferably 97% identity, more preferably 99% identity and particularly 100% identity) with the nucleotide sequence SEQ ID N° 1, provided that said nucleotide sequence comprises the SNP c12972t.

Said Cx26 protein may be the natural Cx26 protein, a Cx26 protein having the same functionality as the natural Cx26 protein and/or a polypeptide having at least 95% (preferably 97% identity, more preferably 99% identity and particularly 100% identity) with the amino acid sequence SEQ ID N° 2.

Preferably, the Cx26 protein is used for the preparation of a medication for the prevention or the treatment of one of the diseases selected from the group consisting of the dominant and recessive deafness, non-syndromic autosomal or syndromic deafness, diffuse keratoderma, mutilating keratoderma, hyperkeratosis, bladder and breast cancers.

## Claims

1. Isolated polynucleotide comprising:
a) a nucleotide sequence having at least 80 % identity with the sequence SEQ ID N° 1 or its coding sequence, it being understood that this nucleotide sequence contains the following coding SNP: c12972t; or
b) a nucleotide sequence complementary to a nucleotide sequence under a).

2. Isolated polynucleotide comprising:
a) the nucleotide sequence SEQ ID N° 1 or its coding sequence, it being understood that each one of these sequences comprises the following coding SNP: c12972t, or
b) a nucleotide sequence complementary to a nucleotide sequence under a).

3. Polynucleotide according to any one of claims 1 to 2, **characterized in that** it consists of the sequence SEQ ID N° 1 or its coding sequence, it being understood that each one of the sequences contains the following coding SNP: c12972t.

4. Isolated polynucleotide consisting of a part of a polynucleotide according to any one of claims 1 to 3, it being understood that the sequence of said isolated polynucleotide contains the following coding SNP: c12972t, said isolated polynucleotide being composed of at least 10 nucleotides

5. Isolated polynucleotide, **characterized in that** it codes for a polypeptide comprising the amino acid sequence SEQ ID N° 2, it being understood that the amino acid sequence contains the following coding SNP: R98W.

6. Polynucleotide according to any one of claims 1 to 5, **characterized in that** it is composed of a DNA or RNA molecule.

7. Use of all or part of a polynucleotide according to any one of claims 1 to 6, to identify, hybridize and/or amplify all or part of a polynucleotide consisting of the sequence SEQ ID N° 1 or of its coding sequence, it being understood that each one of these sequences contains the following SNP: c12972t.

8. Use of all or part of a polynucleotide according to any one of claims 1 to 6, for the genotyping of a nucleotide sequence which has 90 to 100 % identity with the nucleotide sequence of CX26 gene and which comprises at least the following SNP: c12972t.

9. Use according to claim 8, in which the genotyping is carried out by minisequencing.

10. Recombinant vector comprising a polynucleotide according to one of claims 1 to 6.

11. Host cell comprising a recombinant vector according to claim 10.

12. Process for the preparation of a polypeptide, **characterized in that** a host cell according to claim 11 is cultivated in a culture medium and said polypeptide is isolated from the culture medium.

13. Isolated polypeptide comprising an amino acid sequence having at least 80 % identity with the amino acid sequence SEQ ID N° 2, it being understood that the amino acid sequence contains the following coding SNP: R98W.

14. Polypeptide according to claim 13, **characterized in that** it comprises the amino acid sequence SEQ ID N° 2, it being understood that the amino acid sequence contains the following coding SNP: R98W.

15. Polypeptide according to any one of claims 13 and 14, **characterized in that** it consists of the amino acid sequence SEQ ID N° 2, it being understood that the amino acid sequence contains the following coding SNP: R98W.

16. Process for obtaining an immunospecific antibody, **characterized in that** it is obtained by immunization of an animal with a polypeptide according to any one of claims 13 to 15.

17. Immunospecific antibody for a polypeptide according to any one of claims 13 to 15.

18. Process for the identification of an agent activating or inhibiting a polypeptide according to any one of claims 13 to 15, comprising:
a) the preparation of a recombinant vector comprising a polynucleotide according to any one of claims 1 to 6,
b) the preparation of host cells comprising a recombinant vector according to a),
c) the contacting of the host cells according to b) with an agent to be tested, and
d) the determination of the activatory or inhibitory effect generated by the agent to be tested.

19. Process for the identification of an agent activated or inhibited by a polypeptide according to any one of claims 13 to 15, comprising:
a) the preparation of a recombinant vector comprising a polynucleotide according to any one of claims 1 to 6,
b) the preparation of host cells comprising a recombinant vector according to a),
c) the contacting of the host cells according to b) with an agent to be tested, and
d) the determination of the activatory or inhibitory effect generated by said polypeptide on the agent to be tested.

20. Process for the detection of the expression and/or of the activity of a polypeptide of amino acid sequence SEQ ID N°2 encoded by a polynucleotide according to any one of claims 1 to 6 and/or of a polypeptide according to any one of claims 13 to 15, comprising:
a) the detection of the presence or of the absence of a polynucleotide according to any one of claims 1 to 6 in the genome of the subject, and/or
b) the determination of the concentration of a polypeptide according to any one of claims 13 to 15, in a biological sample of the subject.

21. Medication comprising by way of active agent at least one polypeptide according to any one of claims 13 to 15.

22. Use of a polypeptide according to anyone of claims 13 to 15, for the preparation of a medication intended for the prevention or the treatment of one of the diseases selected from the group consisting of the dominant and recessive deafness, non-syndromic autosomal or syndromic deafness, diffuse keratoderma, mutilating keratoderma, hyperkeratosis, bladder and breast cancers.

23. Medication comprising by way of active agent at least one polynucleotide according to any one of claims 1 to 6, a recombinant vector according to claim 10, a host cell according to claim 11 and/or an antibody according to claim 17.

24. Use of a polynucleotide according to any one of claims 1 to 6, a recombinant vector according to claim 10, a host cell according to claim 11 and/or an antibody according to claim 17, for the preparation of a medication intended for the prevention or the treatment of one of the diseases selected from the group consisting of the dominant and recessive deafness, non-syndromic autosomal or syndromic deafness, diffuse keratoderma, mutilating keratoderma, hyperkeratosis, bladder and breast cancers.

25. Pharmaceutical composition containing by way of active agent at least one polypeptide according to any one of claims 13 to 15, all or part of a polynucleotide according to any one of claims 1 to 6, a recombinant vector according to claim 10, a host cell according to claim 11 and/or an antibody according to claim 17, as well as a pharmaceutically acceptable excipient.

26. Diagnostic composition containing by way of active agent at least one polypeptide according to any one of claims 13 to 15, all or part of a polynucleotide according to any one of claims 1 to 6, a recombinant vector according to claim 10, a host cell according to claim 11 and/or an antibody according to claim 17.

27. Use:
a) of a therapeutically effective quantity of a polypeptide according to any one of claims 13 to 15, and/or
b) of a polynucleotide according to any one of claims 1 to 6, and/or
c) of a host cell according to claim 11, this cell coming from the subject to be treated, in order to prepare a medication intended to increase the expression or the activity in a subject, of a polypeptide according to any one of claims 13 to 15.

28. Use:
a) of a therapeutically effective quantity of an immunospecific antibody according to claim 17, and/or
b) of a polynucleotide permitting inhibition of the expression of a polynucleotide according to any one of claims 1 to 6, in order to prepare a medication intended to decrease the expression or the activity, in a subject, of a polypeptide according to any one of claims 13 to 15.

29. Use of a Cx26 protein for the preparation of a medication for the prevention or the treatment of a patient having a disorder or a disease caused by the R98W Cx26 variant linked to the presence in the genome of said patient of a nucleotide sequence having at least 95% identity with the nucleotide sequence SEQ ID N° 1, provided that said nucleotide sequence comprises the SNP c12972t.

30. Use of a Cx26 protein according to claim 29, for the preparation of a medication for the prevention or the treatment of one of the diseases selected from the group consisting of the dominant and recessive deafness, non-syndromic autosomal or syndromic deafness, diffuse keratoderma, mutilating keratoderma, hyperkeratosis, bladder and breast cancers.


LISTE DE SEQUENCES

<110> GENODYSSEE

<120> New polynucleotides and polypeptides of CX26 gene

<130> CX26

<140>
<141>

<160> 4

<170> PatentIn Ver. 2.1

<210> 1
<211> 13980
<212> ADN
<213> Homo sapiens

<400> 1
gaattcacca gtgaagctat ctgggcctgg agttttattt gtgggaaagg tttttgttac 60
aaattcaatt ttcttaatag atacattttt tcatttctgt ttgtgcccat tttgataagt 120
tgtatttttc aaggaattta aatttgttca cttcaatcaa gttattgaat ttattggaag 180
aaaattgttc ctacatttta ttattaactt ttttagtgtc tgtaggatct gttgtgattt 240
cccctctttc attcctgatg acatagttta tgttttttct cttttttcct taattactgt 300
tgcttggaat gttatggaca gaattgtgtc cctccaacaa tcatacatca aagcactact 360
gtgcagtgtg atggtgtttg gagactgagc ctttgggaga tagctaggtt gagatgaggt 420
tatgagggtg tggccctcat gatgggatga gcgcccttat aaaaagagac accagagagc 480
ttgcacctct ctctctctct gccatgtgag cacacagcaa gaaggctacc atctgcaagc 540
caggaagaaa gccctcacca gaacccaagc atactggcat cctgatcttg gacttccagc 600
ctccagaacc atgacaaaat aagtttctgt tgtttaagct acccagtcta tggtattttg 660
ttatggcagc ccaagctaag acaaagagtt tatcaatttt attaatattt tcaaagtatt 720
aaactttggc tttgtttgat ttctctattt agttgccttc tacttaattg atttttttat 780
atttgttatt tcctttctgt acatacaatg agtttcattt gttctttttt cacacttctt 840
aaggtggaaa tgtaatagtc aattctaaat ttttcttctt tttctaatat ttatttttaa 900
tatttaaggc aaaaatttct ctgttagcac tgcttcaggt acatacccca aattttaata 960
tttatgtttt cattttcatt gattttaaac atttcaaatg tctcaaaatt ccttctttta 1020
ctcatgggtt atttgttatt taatttccaa atgtttggta ttttctagat attcatatat 1080
ctagtttaat tctgcaacag tcagagaata tattctgtga gatttctatt ccttggaatt 1140
tattgagaca tttcatagtt cagcatgtct tttctcaaca aacatttcac atgcacttga 1200
aaacgatgtg tattatgcag ttgctaaacc tggtgttcta taaatgtaag tgaggccaaa 1260
ttagctgata gtattcctta gatcttctat atccttactg atttttatta tatacatgtt 1320
ctgtcaatta ctcagttatt ataactatga atatggattt gtcatttttc cctttggtgt 1380
tcttaatatt gctttgtgta ttttggatct ctgttactta gggaatacac atttaagagt 1440
gatgtcttct tctttttttt ttttttttga aatggagtct cgcccagttg cccagactgg 1500
agtgcatggt gcgaactcag ctcactgcaa cctccgccca ctgggttcaa atgaatctcc 1560
ccgtctagct gggattgctg acgcccacca ccacgcccag ctaatttttt gtatttttag 1620
tatagatggg gttttgccat gttagccagg ctggtcttga actcctgacc tcaggcaatc 1680
cgcctgcctg ggcctcccaa agtgcaggga ttactggtgt gagccactgc acctggccaa 1740
gattgatgtc ttcttgatga aaagaccctt ttaccatttg agatgtcttt ttaactctgt 1800
tagtttcctt gttttgaagt atattttgta cagtaaagcc accccagctt ccctataatt 1860
taagtttgca ttgtgtatgt acattttccc atacttttat tttcaatatg tagtgtatct 1920
acttctattg actgtttttt tttttcctcc tgattatgga tctcattttc ctatttcctc 1980
acgtcttgtg atttttttac tgaaggttag acattgcata taaaagactc tccaggagtg 2040
ttttgttttt gttttttcta gtgagtgcaa gtccttttct ctctgccagc tggctggaat 2100
gagaatctga tcagatttca tcaagagtca ggttgagctg agactgagct gtagtgttca 2160
ctaaattgag tgcaccactg atatctaatg gaaacaagga cgttttactt tgctcctcag 2220
cctaacctga atttcctatg ccgccactgt ataatggcta gtttctttgg ttttactact 2280
gttcaaactg gaagcaggtt gagacataga tttcatatca tttggcttac cttgcatcta 2340
acatggctcc aaaattcaag cactatgaaa ttgtttgttt tccagccttg cctccactgc 2400

cacttttgca gtaaaatcag ggacaggggg tgactgttga gccaaactat ttttgcattt 2460
ggtggacttc taaattctaa accatctcca aatcttttgg cagatttttc ttaaaggtac 2520
aaagtctctt cattgataga ggcctgcttc tccaccttcc cacacagaaa tcaggtactt 2580
tccctcaagt aagaaagaag ctgtgggctc tctgctcacc atcacgctca gtaattcagc 2640
tcatcaaagc ttaccctctc cactgttctt tggtaattgc cacaaaaaaa gtatgatttt 2700
cattttatca gggttttttg ttattgttat catacgaata atggtatttt gcattctata 2760
ttttaattgg aagcagaagc catattattg ttagtaacag cgaaatatta gaaataatcc 2820
aaacacccat caaaaaaggg tttttaatta aatgattaca taatcatata aaggaatact 2880
atgaaactgt gtgaaagaat gaagaaactc tttgttcact tatataaaaa gatcccatga 2940
tatgttaaga aaagcaaagt gtggaatagt aggtaaaata ttctatctta tgtgcaaaag 3000
gggaaataaa agtcatcaat attcatgtag attcaattca catatagatt catatcacat 3060
tcctatatat atagaaattc tggaaagaca caaaataaat taataaaagt tgttacttca 3120
ttgtagtttt taaagttttt tgagtcttaa gacttacttt ccacttctgt agaaaggaat 3180
tacaaatcct ttctttatag agctatgtga tgaaataaac ataaagcatt tggcacactt 3240
caggatagca acttgtggat taatgattaa cacagtcacc tttgcaccag attacaccca 3300
gagattcctt catttatatt tatgtggttt tgtgtgtcag ttatgcagtc taactcagtc 3360
attcaactat gttacagctg caacactcta tttttttctt tggtacagga gtcgccctct 3420
tatccactgt ttcatttttg tggttccagt tacctgtagt caaccacagt tggaaaatat 3480
gatagcattt tgagagagag actgcatcca aaaacttata ttacaatata ttgttataca 3540
ttgttataag tgttgtttta ttattcttta ttgttaatct cttaccatta agccttatgg 3600
taggtttgta tgtataggaa aaaacagatt atatagaggg ttcagtacca tctgaggttt 3660
caggcatcac tgggggcttg gaatgtaccc ctcatggata atggggaact accataaatt 3720
gtaagcagag gctgtgtctt tctaaagccc agccacctaa ttacctacat gcaatatcac 3780
gtagcctgta agaataagtg aatgtttccc atcagatggt gactgagatg accaatacca 3840
tcttcaccca tgaaagctat gtgtactaga gccccatatc actaatagca atagtctaat 3900
acgctaaatt ccccgccatc attgattcat ttcaaagcca ataaagagac aatataaacc 3960
attatgttta ctgcctcctc ttttccaaag cataattgtt caatgcatac gtttggcatt 4020
aaaagtctct atctggctct ttaaatatgt aacccattaa aatgactgat ccctgatttc 4080
tgagcatact acatatcaaa gtattattcc tatccttttt tagacaaata attgtattaa 4140
actagtaaaa gacctaacta aatgttgcta ttgcttctca gatcacaaag aggagccctg 4200
ggacgtcctt ttccaagctg aaatgatggt cctaagcagc ccatccacaa ataaatctca 4260
ccaacctggg gtttctggga tcaacggact gtgaacttaa ggcacagcag agctggggct 4320
gctcttaagg ccctgctgtc tctcctctta gtaacaacac catttcacat gaagtgacag 4380
tggtatcttt tgttgccctg gaaatggaat acaacaatgg ctttccaact tttctgtggc 4440
agagacctac agacagaagt acattttaca ctggatccag gacacacatc agtctgaaaa 4500
cacacacatg aaccaaacgt ttcctaaagc attacttatc cttgctaata gcaacacatt 4560
ctcatattct tttatacttc atttaatttc atataaaaaa gaaaaggaaa ggaaagaaat 4620
ctatttctca gcccattaat aaggtcagga gcagcaacac cagactagaa gaaaagctta 4680
cctatagatt tttctgccac ctcttgagtg cgtccagctt tccgacaagt ctcagtgcca 4740
tctactgtgc gctctgggta ttgcaattgc tttttttttt tttttttttt tttttttaga 4800
atgagactaa gtcagagaac acaaagaact tctttcccca cagtggagat ggctctgaaa 4860
gcgtttaagg aatagcttag atgagtggct aacacattct cccggttctg aattctaaga 4920
ccacagactc catgtccagt ccccaaagag aggctttgca agctacagaa tacccctctg 4980
actgggacct caggagctaa actgaccacg taattggttc tagaaagtga aacgttttaa 5040
tttgaaacat ccaaatgagc attttgtgaa aagctactgc cgtccatcaa atacaacaca 5100
gccagggagt catcgctcta ttgcccttgt caatcctaca tctatagttt tttttgctac 5160
agcagttcat gagtgttgac tctattctaa cttgttccag aagcccttca agatgataga 5220
tagcacaata tttttgtagc cagagctaga atgtagagct ctttttggct tccttgtgaa 5280
tgatccagaa tttccatgtt ggcaagccac cataatttac agaatttact ttttatattc 5340
aatagaagta aaaaaaattt acctatttaa ggagttatag ctctggattc atttctgacc 5400
aaaatgtgct ttttgacaca aatacaattg gaaatgtctt tgtaatttat ccacagtctg 5460
cctagataat cataaaagaa ctgcatggat atatttgtga gtaagagcac gtgtccattc 5520
agcaaaacca aggagatcaa ctaattctac cattgccttg aaacggagac acatctagca 5580
gtttgaattt cccccaaaag attgtatgtg tgaaaataag aatagaatga ggaaaattta 5640
aaagcctata taataatttc agtcacaact tggcaattag aattttatga gatgtcttta 5700
atttggaagc aaagaacaat taaattattg aaggctggaa ttttttttta actctttgaa 5760
tggaacaaca gattttcccc aaaagatttg actttaacaa ttttcagaaa acataagtca 5820
gggtgtggtt caattacaca gagagaaatt gtagtgaaat agtgttccct gtaataatta 5880
cccacaaagg agcacagtgg agccactcct gcattaaaat tacagtatca tatgtaagtt 5940
attattaatt aaccagagat gccaggagct tgtcagtttc caactgctat tttgaggaga 6000
gctaaagttt ctcttttttt gccagttatt attattatta atatttcaac agcaaggcaa 6060
gaaaagggaa tgtggtccat taactaatgg ctcttgaaaa gacactcaat gaatccaact 6120
tgccctaaaa ctgccaagtg gtaggacagt ctcttcgcgt cttgcatcat tttctgccat 6180

cacctacgtg tgattcgtga gtcggaaatt caaccaagac atgtttaatg tatatttaga 6240
gcattcttcc cggcgggaat tcacggtgcc attccatcag gcagttggca agcagtcact 6300
tgaaatatta agaaatatga tttgtgtcac actgatttat tgcaaaacag caacttcttt 6360
ctttttggtt catttataaa acaactgtca aattaaaatg ccaaatagct ttaaacatta 6420
gcattttcac cttataacct tacaagtgca tcactttaaa catctgagta aaagttcagc 6480
tcgatgacaa tcacctggga tttacctgca tggtactaag catatatgta aaaatattac 6540
tgatgggtat ctctggcact ctgaagtgac aaagtgtagc cttcacagat ctttgtcagt 6600
taatcatcaa tagttacctg aaaagtgccc acttgccatc attcaagatc aaccaggcag 6660
acaccacagt gagttttcca tcaaaaaacc ttctctatct ggtcagtctc tgcacgtcaa 6720
tgagacaaag gtgtatgctg cacgcagcag tactatccta agctccctgt gtcctcacca 6780
tggggctggg tggctggggt ggaggaacac aggattgggc ttcagcttct ctagggactg 6840
gtacattaag agatgaagac ataaaaggtg agaaaaacat ggtttatttc caatgtttcc 6900
atttctgtta aaagtaatgc tttcaacaga aaaaaaatgc agcaatataa gtgtgtaatt 6960
tacaaaataa tttcaggatt tctttaatca ttaatttgtg gtgtcatctg ttaactggat 7020
ttacgtctaa gctcatttgt aaataacttc aaatatccaa gccttccctc acccttttcc 7080
cacctcacct ctcctccttc tcctccccta cactggagga cactatgtac atgcatataa 7140
tgtcctgccc tagaggagtc ctgagcctac ttgggaagaa aacaccaact cacaggaaaa 7200
cagcagaaat cacacaaaac agaataaaag caagcgctga tctgtaagtg aagacttaag 7260
tgctatagga cttccagcta caaatcctga aaacacggag tggctgtgat aatacgacta 7320
gccaacatca cacagtaatt ttgcacataa ggagaactaa atcaaagaaa acaaggaaaa 7380
gaaagttgag cctataatcg tgatacaggc actaaaatct caggtgacat ttttcaatgg 7440
gggaaagtca gtcaacttcc gatctccaaa ccatctttac tagcgagctt cccacaatgg 7500
ttctagaacc ttccttcatt ccaacccaac caggattcca acagactcat aaacaccaca 7560
gcctttgaga aattaaaggg agaacccacc aaccggcgcc ccactcccca ccccaagtca 7620
cctctggctc aaccaagatg cgctcaggcc aagaaagctg ccccacccca caggctttgc 7680
ctgtcatttt taacaagccg actcagcaca tctctcagat gggccatgca aggcttttcg 7740
cagctcctgg ggctttgcct cttcatgagc agacactccc tcttagacta agacctggag 7800
ctggaaagta ggtggtaacc gcggtacaaa actcacgctc gtccctgcag aaactgccta 7860
ggtcggccca tggccacggg gcgccaattt ttcaaggaaa agtcaatgct aataatggtg 7920
gcaatcacgg gaaatccatt ctgaggccag atctgacttg tcaggattaa tcatcatttc 7980
cacttaactt cgaactgacc tgggtaaaaa cgtgagcgcg aggggaccag gctgcacctc 8040
tgacctggct cccctctgca aaaatcgcga agtgggtgcc cgaggtgggg cgggggttgg 8100
gggagacctc cccgggagtc cccacccagc ctgctctgca catcttagtc cctcatccgc 8160
ttgcgctgtg caaatctgtc ttctgtcatt tgtatcgcaa gacatcaaaa tccccaacca 8220
aatgcaaata ctgagacctc ataatctgag acaaagtttc acggtatcca gaaagccccc 8280
agcaggtgtg cagtgcagag ccagcccccc agcggtcttc cgcagaatcc tatcagtttc 8340
cccctttcgt gctgtgtgca tcgagcagga aggggcttgg caggttttac ctgccctctt 8400
tcctttctga aaagtctggg cctcctcacc ccgaaaggag tcacctcctt gcagttcccc 8460
agttgcgaaa agaggaggaa gttggctggg ccgggggccg cggggggcac cctccgcaga 8520
tggcgggacc cccctgccgg ccatggcaaa aacgaggctt gtctctccca ccgcccccaa 8580
ccttagtcct tggcacattg ttgaaagtaa ttgaataaaa tcggaaattc gagaaggcgt 8640
tcgttcggat tggtgagatt ttgaggggag aaagaagcgg ggacttcgcc ggcaccagcg 8700
gcgccccctc ctcggccacc gttaaccccc attccagagg gcactgcccc gccacccagc 8760
ctaggtcccc ctgcgagagc ctcgcgggcc cgcgcagcct ccgcgactcg aacagatctt 8820
cagtccttgg aggaatgcct gtttctctaa caataaaaaa ttaaagaagc gctcataaat 8880
gccaagtcct ctcgcactat gcggagtaca gaggacaacg accacagcca tccctgaacc 8940
ccgcccacgg cacagcgccg gagccggggt ctggggcgcc gcttcctggg gggtcccgac 9000
tctcagccgc ccccgcttca cccgggccgc caaggggctg ggggaggcgg cgctcggggt 9060
aaccggggga gactcagggc gctgggggca cttggggaac tcatgggggc tcaaaggaac 9120
taggagatcg ggacctcgaa ggggacttgg ggggttcggg gctttcgggg gcggtcgggg 9180
gttcgcggac ccgggaagct ctgaggaccc agaggccggg cgcgctccgc ccgcggcgcc 9240
gccccctccg taactttccc agtctccgag ggaagaggcg gggtgtgggg tgcggttaaa 9300
aggcgccacg gcgggagaca ggtgttgcgg ccccgcagcg cccgcgcgct cctctccccg 9360
actcggagcc cctcggcggc gcccggccca ggacccgcct aggagcgcag gagccccagc 9420
gcagagaccc caacgccgag acccccgccc cggccccgcc gcgcttcctc ccgacgcagg 9480
tgagcccgcc ggccccggac tgcccggcca ggaacctggc gcggggaggg accgcgagac 9540
ccagagcggt tgcccggccg cgtgggtctc ggggaaccgg ggggctggac caacacacgt 9600
ccttgggccg gggggcgggg gccgccttct ggagcgggcg tttctgcggc cgagctccgg 9660
agctggaatg gggcggccgg ggaagtggac gcgatggcac cgcccggggt gcgagtgggg 9720
ccgggcgcgc gcgggagggg aaaaaggcgc gggcgagccg ccagcgcgag gtttgtggtg 9780
tcgccgatgt cccttcgggg tactctagcg cagccgcctg gctacttgac ccactgccac 9840
caaacgtttt aaattcaccg aaagcttagc ttcgaagcaa agctccgttt cgccggtgaa 9900
gcaggaagcc ttcgctgcag gaactgacct ttacctcttg gagcggcttc tgcagaaaaa 9960

tccccgggca gagatttggg cggagtttgc ctagaactaa cgcggagcca gccgatcccg 10020
gcctaccccg gggccaagat ttcagtggct tccctttttc ctaaacactt cacgagggtc 10080
tgtttccggg ctgtgctccc cgcctagaag gaaaattttt aggacccttg ttcgcgaaga 10140
ggtggtgtgc ggctgagacc cgcgtcctca ggacggttcc atcagtgcct cgatcctgcc 10200
ccactggagg aggaaggcag cccgaacagc gctcacctaa ctaacagctg ctgagagctg 10260
ggttccgtgg ccatgcacct gggactgcct tgagaagcgt ggtacggccg tgtccccatg 10320
tgaccttaga gtccctttcg aaactgctgt gcacagtcgg tcacaatttc agacactggt 10380
gagaagggtg gaggaaccct ctggggacag ccaggcaagg tcgaccaccc atcacctaag 10440
ggtggagaaa tttaaggggt gaagagtccc ttttgccttt tctggatcct ggtgattcac 10500
ctagtgtctt ccctaaggaa ctgaaccaac tcctccgctg gcctctggca gccctccagg 10560
cggtgcagga tggcgtgggc ccggtaggaa gctgcatgta accgcccagg gtcgggaggc 10620
caggagggca gctcctcctc tgacttgaat attgaaaaca acttcgtcct gcttctgagc 10680
ccctcttaac ccatgacccc ctagcccatt ggggagtaaa tcttaattta ctcctcttcc 10740
tgaaaaagga tctttaaaac aggtagcttc aactcaagct ttataaaata acaatatagg 10800
gtttctcgga actgtatttt tctcagctga tggtaactgg acaggtctgt agaagggtgt 10860
atgacctggg tttggcaggt ggaagagggc aaaggataaa cccctcctcc tgcagcccca 10920
tattcttggc caggtgtatt gttgtaaacc aggagagagt ttacttcggg gagtatcctg 10980
ttttccactc agtgagggcc aatgaagaat gtctaattcc ataagatgct tttgttaaaa 11040
tcggaatgtt gctgtcctcg gtggttctgc tgttgggacg ggactggcct gagctgtggg 11100
tgctgtagca ggacaaccag ctcacctaag ggcctcccag tctggattat caatgggtca 11160
gtgctgaacc tgggctaaaa tattgttttt tccaatgatg ttgtctttcc caagctcagt 11220
gaagctaaat gtttcacagg cctatgtcaa tctgatgtaa ctttcgtggc cacctctctc 11280
ctgttagcct ctgaccaagg tggcactgga tggtttctgc ctgaccttgg tgccccgtgg 11340
cagcgactgt gggtcatgaa agacattcac tacgagcctg cttctggagt ccatcagaaa 11400
acggatgcaa acttgcctaa aatgaggaga ggaggatgct tttaagaaaa agaagaagga 11460
ggattcacta ccagctctga agggtggaaa agagatgatt catccggatt gtggagaggg 11520
tggaatcttg tttaggagag cgttggttgt ggcaggcagg gtgtaactat gaatcagtga 11580
agacaattca catcctggga tgaaaagaag gccatgggct cacaggagat tatccactgg 11640
cctctccaca tccgcttgca gtaaggagtg tgggactctc ccaagcttca gcgctgaact 11700
gcaatgcagt gacgtcgctt agctgggcca gtaaccgagg gagttgaatt ttctgtcatt 11760
ttaaaataat gtgtctttta agaaacactt tgaaattaaa accacagccc acaattataa 11820
tgcactgttg cagcacttat caaaacagat atgctaactg agccatcagt gccagcctga 11880
cagtgaggcc accaagccat ccacaaagcc tacacgaaag tctgtgctca cagtggcttt 11940
tctccatgaa gagggcattc ctaacctctt cctttcacgt aggaggaagc aaggtccttt 12000
gtaaaatttt aactcggggt gcctcaaatg taaacttaac cactggtaac aacagtttca 12060
ctgctacatg ccacgtctgt gaaaattcat tcaagacatt aaggaaagtg gctcagcaga 12120
gagactagac atcttatcct cacggttctc ctgtacttgg cctctcagcc tttgagcaag 12180
gttggcccaa gctagtatcg gccccagtgg tacagccaaa acttgagact gcaaatggat 12240
gcagctgttg aacgctgagt aacttctgca gagtcaggaa gacccaagga agctctgcag 12300
aggatgcagg ggtacggtca gaacccctga gtgcctttca gctaacgagg actttatgac 12360
actccccagc acagcaaatt tttatgatgt gtttaaagat tgggtgaatt actcaggtga 12420
acaagctact ttttatcaga gaacacctaa aaacacgttc aagagggttt gggaactata 12480
catttaatcc tatgacaaac taagttggtt ctgtcttcac ctgttttggt gaggttgtgt 12540
aagagttggt gtttgctcag gaagagattt aagcatgctt gcttacccag actcagagaa 12600
gtctccctgt tctgtcctag ctagtgattc ctgtgttgtg tgcattcgtc ttttccagag 12660
caaaccgccc agagtagaag atggattggg gcacgctgca gacgatcctg gggggtgtga 12720
acaaacactc caccagcatt ggaaagatct ggctcaccgt cctcttcatt tttcgcatta 12780
tgatcctcgt tgtggctgca aaggaggtgt ggggagatga gcaggccgac tttgtctgca 12840
acaccctgca gccaggctgc aagaacgtgt gctacgatca ctacttcccc atctcccaca 12900
tccggctatg ggccctgcag ctgatcttcg tgtccacgcc agcgctccta gtggccatgc 12960
acgtggccta ccggagacat gagaagaaga ggaagttcat caagggggag ataaagagtg 13020
aatttaagga catcgaggag atcaaaaccc agaaggtccg catcgaaggc tccctgtggt 13080
ggacctacac aagcagcatc ttcttccggg tcatcttcga agccgccttc atgtacgtct 13140
tctatgtcat gtacgacggc ttctccatgc agcggctggt gaagtgcaac gcctggcctt 13200
gtcccaacac tgtggactgc tttgtgtccc ggcccacgga gaagactgtc ttcacagtgt 13260
tcatgattgc agtgtctgga atttgcatcc tgctgaatgt cactgaattg tgttatttgc 13320
taattagata ttgttctggg aagtcaaaaa agccagttta acgcattgcc cagttgttag 13380
attaagaaat agacagcatg agagggatga ggcaacccgt gctcagctgt caaggctcag 13440
tcgctagcat ttcccaacac aaagattctg accttaaatg caaccatttg aaacccctgt 13500
aggcctcagg tgaaactcca gatgccacaa tggagctctg ctcccctaaa gcctcaaaac 13560
aaaggcctaa ttctatgcct gtcttaattt tctttcactt aagttagttc cactgagacc 13620
ccaggctgtt aggggttatt ggtgtaaggt actttcatat tttaaacaga ggatatcggc 13680
atttgtttct ttctctgagg acaagagaaa aaagccaggt tccacagagg acacagagaa 13740

ggtttgggtg tcctcctggg gttctttttg ccaactttcc ccacgttaaa ggtgaacatt 13800
ggttctttca tttgctttgg aagttttaat ctctaacagt ggacaaagtt accagtgcct 13860
taaactctgt tacacttttt ggaagtgaaa actttgtagt atgataggtt attttgatgt 13920
aaagatgttc tggataccat tatatgttcc ccctgtttca gaggctcaga ttgtaatatg 13980

<210> 2
<211> 226
<212> PRT
<213> Homo sapiens

<400> 2
Met Asp Trp Gly Thr Leu Gln Thr Ile Leu Gly Gly Val Asn Lys His
1               5                   10                  15

Ser Thr Ser Ile Gly Lys Ile Trp Leu Thr Val Leu Phe Ile Phe Arg
            20                  25                  30

Ile Met Ile Leu Val Val Ala Ala Lys Glu Val Trp Gly Asp Glu Gln
        35                  40                  45

Ala Asp Phe Val Cys Asn Thr Leu Gln Pro Gly Cys Lys Asn Val Cys
    50                  55                  60

Tyr Asp His Tyr Phe Pro Ile Ser His Ile Arg Leu Trp Ala Leu Gln
65                  70                  75                  80

Leu Ile Phe Val Ser Ser Pro Ala Leu Leu Val Ala Met His Val Ala
                85                  90                  95

Tyr Arg Arg His Glu Lys Lys Arg Lys Phe Ile Lys Gly Glu Ile Lys
            100                 105                 110

Ser Glu Phe Lys Asp Ile Glu Glu Ile Lys Thr Gln Lys Val Arg Ile
        115                 120                 125

Glu Gly Ser Leu Trp Trp Thr Tyr Thr Ser Ser Ile Phe Phe Arg Val
    130                 135                 140

Ile Phe Glu Ala Ala Phe Met Tyr Val Phe Tyr Val Met Tyr Asp Gly
145                 150                 155                 160

Phe Ser Met Gln Arg Leu Val Lys Cys Asn Ala Trp Pro Cys Pro Asn
                165                 170                 175

Thr Val Asp Cys Phe Val Ser Arg Pro Thr Glu Lys Thr Val Phe Thr
            180                 185                 190

Val Phe Met Ile Ala Val Ser Gly Ile Cys Ile Leu Leu Asn Val Thr
        195                 200                 205

Glu Leu Cys Tyr Leu Leu Ile Arg Tyr Cys Ser Gly Lys Ser Lys Lys
    210                 215                 220

Pro Val
225

<210> 3
<211> 18
<212> ADN
<213> Homo sapiens

<400> 3
aaaccgccca gagtagaa                                                    18


<210> 4
<211> 20
<212> ADN
<213> Homo sapiens

<400> 4
cccttgatga acttcctctt                                                  20